# EUROPEAN PATENT APPLICATION

(11) **EP 1 923 432 A1**
(43) Date of publication of application: **21.05.2008**
(21) Application number: 06782521.6
(22) Date of filing: 09.08.2006
(51) Int. Cl.: C09B 57/10, C07D 495/04, G01N 33/53, G01N 33/532, C07F 3/06, C12Q 1/28

(54) **METHOD OF STAINING SUBSTANCE HAVING PHOSPHATE GROUP**

(30) Priority: 09.08.2005 JP 2005230583
(71) Applicant: Manac Inc., Fukuyama-shi, Hiroshima 721-0956 (JP)
(72) Inventor: KOIKE, Tohru, Hiroshima-shi, Hiroshima 732-0063 (JP); KINOSHITA, Eiji, Hiroshima-shi, Hiroshima 732-0066 (JP); KINOSHITA, Emiko, Hiroshima-shi, Hiroshima 732-0066 (JP)
(74) Representative: HOFFMANN EITLE
(86) International application number: PCT/JP2006/315705
(87) International publication number: WO 2007/018225

(57) **Abstract**

A method enabling a compound having a phosphate group to be easily detected in a biological sample and the like, a method for conveniently identifying a phosphorylated amino acid residue, and a compound able to be used in the method as a result of having a high ability to coordinate bond with a substance having a phosphate group are provided.

A metal complex compound represented by general formula (I): 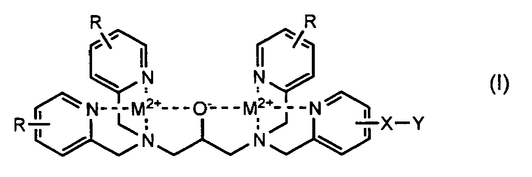 (wherein, M represents a metal atom capable of becoming a dication, each R may be identical or different from each other and represents either a hydrogen atom; an alkyl group of 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group (wherein, the alkyl part thereof is of 1 to 16 carbon atoms); a carboxyl group; a carbamoyl group; a cyano group; a hydroxyl group; an amino group; or a halogeno group, X represents a binding group, and Y represents a labeling group) enables a substance having a phosphate group to be easily identified as a result of having a high ability to coordinate bond with a substance having a phosphate group and having a labeling group.

## Description

### TECHNICAL FIELD

The present invention relates to a method for staining substances having a phosphate group.

### BACKGROUND ART

Certain types of enzymes in the body have serine, threonine, or tyrosine residues at specific sites typically exemplified by active centers or allosteric sites, and the hydroxyl groups thereof are phosphorylated or dephosphorylated by enzymes referred to as kinases and the like, thereby regulating enzyme activity. In addition, there are also enzymes of which activity is regulated by phosphorylation (or dephosphorylation) of the amino or imino groups of lysine, arginine, or histidine or the carboxyl groups of aspartic acid or glutamic acid. The inhibition of glycogen synthesis and its decomposition system are well-known examples of a metabolic system that is regulated by this type of phosphorylation-dephosphorylation. This metabolic system is mainly cascade-regulated and -controlled by phosphorylation-dephosphorylation.

This phosphorylation-dephosphorylation has recently been demonstrated to play an important role in metabolic systems relating to disease. For example, abnormal phosphorylation-dephosphorylation is said to be a factor in cell canceration. In other words, progression and stoppage of the cell cycle is controlled by the phosphorylation (or dephosphorylation) of various enzymes (proteins), and since cyclin and cyclin-dependent kinase (CDK) are involved in this phosphorylation (or dephosphorylation), disturbances occur in phosphorylation (or dephosphorylation) when this mechanism is damaged, thereby triggering abnormal cell proliferation. In addition, protein kinase C has been determined to be related to degranulation of histamine, which causes atopic dermatitis and allergic diseases such as pollenosis, while the neurofibrillary changes occurring in the brains of patients with Alzheimer's disease have been found to be attributable to a phosphorylated protein. Thus, determination of the phosphorylated-dephosphorylated state of proteins has the potential for being useful in not only searching for gene expression of living tissue cells or evaluating enzyme activity, but also in diagnosing and treating diseases.

However, conventionally used methods for identifying phosphorylated proteins (or dephosphorylated proteins) have various shortcomings. For example, although enzyme immunoassays have the advantage of enabling analysis to be performed with only a small amount of target protein sample, it is difficult to obtain an adequate amount of the required antibody, and in the case the target protein has several kilodaltons or less, an antibody that binds to the phosphorylation site in the protein cannot be prepared. In addition, although a method has been conceived for detecting specific binding to protein by using phosphoric acid labeled with radioisotope ³²P, precautions naturally must be taken with respect to the handling of radioisotopes, and those precautions are required to include storage and treatment of waste liquids.

A zinc complex is described in Non-Patent Document 1. This zinc complex has a cleavage function by allowing two zinc ions to act on a phosphate group (phosphate diester group) in a dinucleotide. However, the function of this complex described in this document is only catalytic, and there is no description whatsoever regarding the ability to coordinate bond with a phosphate group. According to a study conducted by the inventors of the present invention, the dissociation constant of this complex with a phosphate group (phosphate diester group) between two nucleotides is actually extremely high. Namely, the ability of this complex to coordinate bond with a phosphate diester group is low.

In addition, Non-Patent Document 2 also describes an iron complex having a structure similar to that of the aforementioned zinc complex. However, this complex was synthesized as a model of the oxygen molecule transport protein, hemerythrin, and the absence of any description or suggestion of the coordinate bonding ability of this complex with phosphate monoester groups is the same as in the case of the aforementioned Non-Patent Document 1.

On the other hand, Patent Document 1 and Non-Patent Document 3 describe a zinc complex capable of capturing substances having a phosphate group and having the structure of an alkoxide-bridged dinuclear zinc complex. In addition, Patent Document 2 describes a method for labeling phosphorylated peptides. However, the former zinc complex alone is able to capture a substance having a phosphate group, but cannot label it. Moreover, examples of the latter phosphorylated peptide labeling method include gel staining, electron spin resonance (ESR) and affinity spin column chromatography, while examples of labeling groups for zinc complex compounds used in the phosphorylated peptide labeling method include fluorescent labeling groups, nitroxide radical-containing groups, and biotin, and since the metal atom of the complex compound used in the phosphorylated peptide labeling method is limited to a zinc atom, there are no specific descriptions of other labeling methods and complex compounds. In addition, although Patent Document 3 and Non-Patent Document 4 describe a method for detecting phosphorylated peptide or phosphorylated protein by surface plasmon resonance (SPR) using a zinc complex having biotin, there are no specific descriptions of other detection methods.
Patent Document 1: International Publication No. WO 03/053932
Patent Document 2: International Publication No. WO 2004/078724
Patent Document 3: International Publication No. WO 2005/038442
Non-Patent Document 1: Morio Yashiro, et al.: "Preparation and Study of Dinuclear Zinc (II) Complex for the Efficient Hydrolysis of the Phosphodiester Linkage in a Diribonucleotide", Journal of the Chemical Society, Chemical Communications, 1995, p. 1793-1794
Non-Patent Document 2: Hidekazu Arii, et al.: "A novel diiron complex as a functional model for hemerythrin", Journal of Inorganic Biochemistry, 2000, Vol. 82, p. 153-162
Non-Patent Document 3: Eiji Kinoshita, et al.: "Recognition of phosphate monoester dianion by an alkoxide-bridged dinuclear zinc (II) complex", Dalton Transactions, 2004, p. 1189-1193
Non-Patent Document 4: Kazuki Inamori, et al.: "Detection and Quantification of On-Chip Phosphorylated Peptides by Surface Plasmon Resonance Imaging Techniques Using a Phosphate Capture Molecule", Analytical Chemistry, 2005, Vol. 77, p. 3979-3985

### DISCLOSURE OF THE INVENTION

### PROBLEMS TO BE SOLVED BY THE INVENTION

With the foregoing in view, an object to be solved by the present invention is to provide a method for easily detecting a substance having a phosphate group by staining that substance, and a method for identifying a phosphorylated amino acid residue by using that staining method. In addition, an object of the present invention is to provide a compound able to be used in the aforementioned method having a superior ability to coordinate bond with substances having a phosphate group.

### MEANS FOR SOLVING THE PROBLEMS

As a result of conducting extensive research on metal complexes capable of coordinating with a phosphate group (phosphate monoester group) in a substance having a phosphate group in order to solve the aforementioned problems, the inventors of the present invention found that a compound of the present invention has an extremely high ability to coordinate bond with phosphate ions or two hydroxyl groups of a phosphate monoester, and as a result thereof, is able to form a complex by strongly coordinating with a phosphate group (phosphate monoester group) in a substance having a phosphate group and by specifically binding to substances having a phosphate group even in a mixed sample containing a large number of substances, and that a metal complex compound of the present invention is able to easily identify that complex as a result of having a labeling group, thereby leading to completion of the present invention.

Namely, the present invention relates to: (1) a method for staining a substance having a phosphate group, characterized by using a metal complex compound represented by general formula (I):

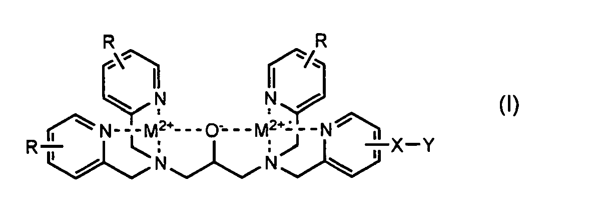

(wherein, M represents a metal atom capable of becoming a dication, each R may be identical or different from each other and represents either a hydrogen atom; an alkyl group of 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group (wherein, the alkyl part thereof is of 1 to 16 carbon atoms); a carboxyl group; a carbamoyl group; a cyano group; a hydroxyl group; an amino group; or a halogeno group, X represents a binding group, and Y represents a labeling group);

(2) a method for staining a substance having a phosphate group, characterized by using a zinc complex compound represented by general formula (II):

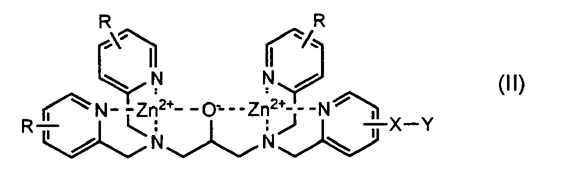

(wherein, each R may be identical or different from each other and represents either a hydrogen atom; an alkyl group of 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group (wherein, the alkyl part thereof is of 1 to 16 carbon atoms); a carboxyl group; a carbamoyl group; a cyano group; a hydroxyl group; an amino group; or a halogeno group, X represents a binding group, and Y represents a labeling group);

(3) the method for staining a substance having a phosphate group described in (1) or (2) above, wherein the labeling group is biotin;

(4) a method for identifying a phosphorylated amino acid residue, characterized by combining the method for staining a substance having a phosphate group described in (1) above with an immunostaining assay using an anti-phosphorylated amino acid antibody;

(5) a method for identifying a phosphorylated amino acid residue, characterized by combining the method for staining a substance having a phosphate group described in (2) above with an immunostaining assay using an anti-phosphorylated amino acid antibody;

(6) a method for identifying a phosphorylated amino acid residue, characterized by combining the method for staining a substance having a phosphate group described in (3) above with an immunostaining assay using an anti-phosphorylated amino acid antibody;

(7) a metal complex compound represented by general formula (III):

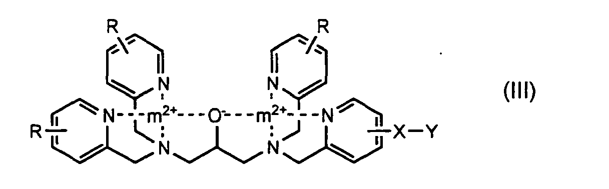

(wherein, m represents a metal atom capable of becoming a dication (excluding the case in which m is a zinc atom), each R may be identical or different from each other and represents either a hydrogen atom; an alkyl group of 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group (wherein, the alkyl part thereof is of 1 to 16 carbon atoms); a carboxyl group; a carbamoyl group; a cyano group; a hydroxyl group; an amino group; or a halogeno group, X represents a binding group, and Y represents a labeling group);

(8) the metal complex compound described in (7) above, wherein the labeling group is biotin;

(9) a staining agent or a staining assistant for a substance having a phosphate group, characterized by comprising the metal complex compound represented by general formula (I) described in (1) above;

(10)a staining kit for a substance having a phosphate group, characterized by comprising the metal complex compound represented by general formula (I) described in (1) above (wherein, Y is an enzyme), a coloring agent for the enzyme, and a substrate as necessary; and

(11)a staining kit for a substance having a phosphate group, characterized by comprising the metal complex compound represented by general formula (I) described in (1) above (wherein, Y is biotin); an enzyme bound by avidin, streptoavidin, or anti-biotin antibody; a coloring agent for the enzyme; and a substrate as necessary.

### EFFECTS OF THE INVENTION

A metal complex compound represented by the aforementioned general formula (I) is able to form a complex by specifically binding to a substance having a phosphate group and has a labeling group, is able to easily identify the complex compound, and is extremely useful for biochemical research and diagnosis and treatment of diseases in particular.

In addition, since the zinc complex compound represented by the aforementioned general formula (II) is able to form a complex by specifically and strongly binding to a substance having a phosphate group under neutral conditions and has a labeling group, is able to easily identify the complex compound, and is extremely useful for biochemical research and diagnosis and treatment of diseases carried out under neutral conditions that are physiological conditions in particular.

In addition, since the complex compound in which the labeling group is biotin in the aforementioned general formula (I) or general formula (II) can be handled easily and applied to various staining methods, it has a high degree of convenience and is able to easily identify substances having a phosphate group.

In addition, since an immunostaining assay using an anti-phosphorylated amino acid antibody is a generally commonly used staining method, combining with a staining method for substances having a phosphate group that uses the metal complex compound represented by the aforementioned general formula (I) makes it possible to easily identify phosphorylated amino acid residues.

In addition, combining a staining method for a substance having a phosphate group that uses the zinc complex compound represented by the aforementioned general formula (II) with an immunostaining assay that uses an anti-phosphorylated amino acid antibody makes it possible to easily identify phosphorylated amino acid residues under neutral conditions that are physiological conditions.

In addition, combining a staining method for a substance having a phosphate group that uses a complex compound in which the labeling group is biotin in the aforementioned general formula (I) or general formula (II) with an immunostaining assay that uses an anti-phosphorylated amino acid antibody is highly convenient and makes it possible to easily identify phosphorylated amino acid residues.

In addition, since the metal complex compound represented by the aforementioned general formula (III) exhibits the ability to coordinate bond with a substance having a phosphate group not found in the prior art, it is useful as a compound that can be used in the aforementioned methods.

In addition, since the metal complex compound in which the labeling group is biotin in the aforementioned general formula (III) can be handled easily and can be applied to various staining methods, it has a high degree of convenience. Moreover, since this metal complex compound exhibits the ability to coordinate bond with a substance having a phosphate group not found in the prior art, it is useful as a compound that can be used in the aforementioned methods.

### BRIEF DESCRIPTION OF THE DRAWINGS

[FIG. 1] FIG. 1 indicates a polyvinylidene difluoride (PVDF) film (A) following staining with a zinc complex compound-horseradish peroxidase (HRP)-bound streptoavidin complex solution, and a gel (B) following staining with SYPRO (registered trademark) Ruby protein gel stain, in Example 2.
[FIG. 2] FIG. 2 indicates a polyvinylidene difluoride (PVDF) film (A) following staining with a zinc complex compound-horseradish peroxidase (HRP)-bound streptoavidin complex solution, and a gel (B) following staining with SYPRO (registered trademark) Ruby protein gel stain, in Example 3.

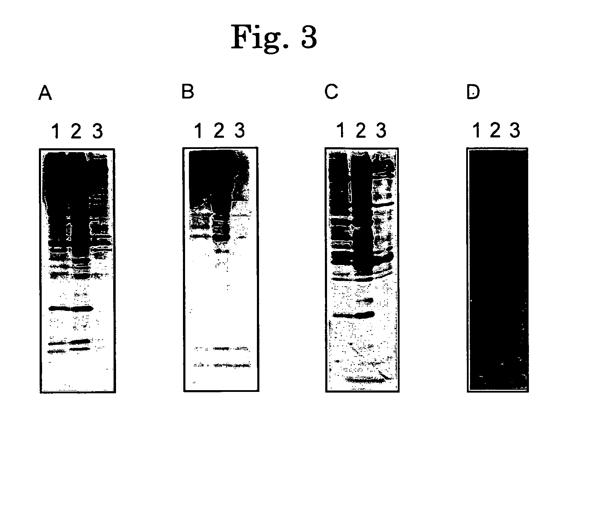

[FIG. 3] FIG. 3 indicates a polyvinylidene difluoride (PVDF) film (A) following staining with a zinc complex compound-horseradish peroxidase (HRP)-bound streptoavidin complex solution, a polyvinylidene difluoride (PVDF) film (B) following staining with a horseradish peroxidase (HRP)-bound anti-phosphorylated tyrosine antibody solution, a polyvinylidene difluoride (PVDF) film (C) following staining with a rabbit anti-phosphorylated serine antibody solution and a horseradish peroxidase (HRP)-bound anti-rabbit immunoglobulin G (IgG) antibody solution, and a gel (D) following staining with SYPRO (registered trademark) Ruby protein gel stain, in Example 4.

### BEST MODE FOR CARRYING OUT THE INVENTION

The foremost characteristic of the present invention is the ability to easily identify a substance having a phosphate group by allowing a metal complex compound represented by general formula (I) or a zinc complex compound represented by general formula (II) having a labeling group to specifically bond with a substance having a phosphate group to form a complex. Namely, although various metal complexes capable of binding with a substance having a phosphate group were known in the prior art, since there were no compounds resembling the metal complex compound represented by general formula (I) or the zinc complex compound represented by general formula (II) and also having a labeling group, the inventors of the present invention found that by using the metal complex compound represented by general formula (I) or the zinc complex compound represented by general formula (II), a substance having a phosphate group can be detected and identified extremely easily even in samples containing a plurality of substances, thereby leading to completion of the present invention.

The following provides an explanation of the metal complex compound represented by general formula (I), the zinc complex compound represented by general formula (II), and the metal complex compound represented by general formula (III).

A "metal atom capable of becoming a dication" is a metal atom of a typical element or transition element capable of becoming an ion having a divalent positive charge. Examples of such metals capable of becoming a dication include beryllium, magnesium, calcium, titanium, vanadium, chromium, manganese, iron, cobalt, nickel, copper, zinc, germanium, strontium, zirconium, niobium, molybdenum, ruthenium, rhodium, palladium, silver, cadmium, tin, barium, tungsten, rhenium, osmium, iridium, platinum, mercury, lead, polonium and radium, with manganese, iron, cobalt, nickel, copper, and zinc being preferable and manganese, iron and zinc being more preferable since the ability of the resulting complex compound to coordinate bond with a substance having a phosphate group is large.

An "alkyl group of 1 to 16 carbon atoms" refers to a linear or branched alkyl group of 1 to 16 carbon atoms, examples of which include a methyl group, an ethyl group, a propyl group, a butyl group, a pentyl group, a hexyl group, a heptyl group, a octyl group, a nonyl group, a decyl group, an undecyl group, a dodecyl group, a tridecyl group, a tetradecyl group, a pentadecyl group, a hexadecyl group, an isopropyl group, and a t-butyl group, with linear or branched alkyl groups of 1 to 4 carbon atoms being preferable, and alkyl groups of 1 or 2 carbon atoms being more preferable.

The alkyl part of "an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group" is the same as previously defined.

A "binding group" refers to any group that binds a main backbone and a labeling group, and has the action of facilitating the production of the complex compound or preventing inhibition by the labeling group of coordination between the complex compound and a phosphate group in a substance having a phosphate group. Thus, in the production of the complex compound, in the case of a raw material compound in which a labeling group is bound directly to a main backbone being easily acquired or a labeling group being comparatively small so as not to inhibit coordination to a phosphate group, the binding group may simply be a covalent bond that directly binds the main backbone and the labeling group. Although there are no particular limitations on the binding group provided it has the action described above, examples include a C1-C6 alkylene group, an amino group (-NH-), an ether group (-O-), a thioether group (-S-), a carbonyl group (-C(=O)-), a thionyl group (-C(=S)-), an ester group, an amido group, an urea group (-NHC(=O)-NH-), or a thiourea group (-NHC(=S)-NH-); a C1-C6 alkylene group having on one end thereof a group selected from the group consisting of an amino group, an ether group, a thioether group, a carbonyl group, a thionyl group, an ester group, an amido group, an urea group, and a thiourea group; a C1-C6 alkylene group having on both ends thereof the identical or different groups selected from the group consisting of an amino group, an ether group, a thioether group, a carbonyl group, a thionyl group, an ester group, an amido group, an urea group, and a thiourea group; and a group in which two or more, preferably 2 to 12 and particularly 2 to 6 groups are linearly bound, said groups being selected from the group consisting of an amino group, an ether group, a thioether group, a carbonyl group, a thionyl group, an ester group, an amido group, an urea group, a thiourea group, and C1-C6 alkylene groups. Here, a C1-C6 alkylene group refers to a linear or branched divalent aliphatic hydrocarbon group of 1 to 6 carbon atoms, examples of which include methylene, ethylene, propylene, tetramethylene, hexamethylene, ethylmethylene, methylpropylene, and dimethylpropylene, with C1-C4 alkylene groups being preferable and C1-C2 alkylene groups being more preferable.

Although there are no particular limitations on the "labeling group" provided it is used in immunostaining assays and staining methods similar thereto, in terms of handling ease, those containing a radioisotope element are not preferable. Examples of such labeling groups include enzymes, fluorescent labeling groups, biotin, and gold colloids. Although the labeling group is introduced into the complex compound by means of a binding group, the method thereof is known among persons with ordinary skill in the art.

Enzymes are proteins having catalytic activity, and enzymes typically used as labeling enzymes in the field of biochemistry can be used without any particular limitations. Examples of such enzymes include peroxidases such as horseradish peroxidase (HRP), alkaline phosphatase, and luciferases. A substance having a phosphate group that has been labeled with enzyme can be stained and detected by allowing a coloring agent or a coloring agent with a substrate corresponding to each enzyme to act. Examples of such coloring agents or combinations of coloring agents and substrates for these enzymes include ECL (trademark), Super Signal (registered trademark), or combination of diaminobenzidine (DAB) and peroxidase for horseradish peroxidase (HRP); CDP-Star (trademark), CSPD (registered trademark), combination of nitro blue tetrazolium (NBT) and 5-bromo-4-chloro-3-indolyl phosphate, or AttoPhos (trademark) for alkaline phosphatase; and luciferin for luciferase.

Fluorescent labeling groups refer to substituents able to stably emit comparatively long-wavelength fluorescence, and those, both water-soluble and lipid-soluble, typically used in the field of biochemistry can be used without any particular limitations. Examples of such fluorescent labeling groups include aminomethylcoumarin and derivatives thereof, fluorescein and derivatives thereof, tetramethylrhodamine and derivatives thereof, anthraniloyl and derivatives thereof, nitrobenzoxadiazole and derivatives thereof, and dimethylaminonaphthalene and derivatives thereof.

Biotin has specific and strong affinity for ovalbumin-derived avidin and actinomycetes-derived streptoavidin. Thus, the complex compound of the present invention can be specifically bound to an enzyme by means of biotin and avidin, streptoavidin, or an anti-biotin antibody by causing avidin, streptoavidin, or the anti-biotin antibody to bind to the complex compound having biotin as a labeling group and further allowing the biotinated enzyme to act thereon, or by allowing an enzyme bound with avidin, streptoavidin, or the anti-biotin antibody to act thereon. A substance having a phosphate group can then be identified by using peroxidase, alkaline phosphatase, or luciferase and the like for the enzyme and allowing a coloring agent corresponding to the enzyme to act thereon. For example, if the complex compound having biotin as a labeling group is bound to a substance having a phosphate group, and an enzyme being alkaline phosphatase is bound to the complex compound by means of streptoavidin, and allowed to react for several hours using coloring agents in the form of nitro blue tetrazolium and 5-bromo-4-chloro-3-indolyl phosphate, since the substance having a phosphate group is stained violet, the substance can be identified on the basis thereof. In addition, streptoavidins labeled with a fluorescent labeling group such as rhodamine are also available commercially, and the use thereof allows a substance having a phosphate group to be identified using ordinary fluorescent image analysis.

Gold colloids have aggregates of gold atoms dispersed in a liquid phase and the like, and can be observed directly with a scanning electron microscope or transmission electron microscope. Thus, the presence of a substance having a phosphate group labeled with gold colloid can be observed with an electron microscope. In addition, it can also be used for immunohistological staining with a transfer membrane or light microscope by a sensitization method using a silver reagent.

The following provides an explanation of a staining method for a substance having a phosphate group.

A "substance having a phosphate group" refers to a substance having a phosphate monoester dianion (-OPO₃²⁻), namely a substance having a phosphate monoester group. Examples of such substances having a phosphate group include phosphorylated amino acids, phosphorylated amino acid residues, proteins having phosphorylated amino acid residues (phosphorylated proteins), polypeptides having phosphorylated amino acid residues (phosphorylated polypeptides), oligopeptides having phosphorylated amino acid residues (phosphorylated oligopeptides), peptides having phosphorylated amino acid residues (phosphorylated peptides), deoxyribonucleic acid (DNA), ribonucleic acid (RNA), phospholipids, and phosphorylated saccharides.

A "staining method" mainly refers to an immunostaining assay and staining methods similar thereto. The immunostaining assay refers to a method for detecting a certain molecule by carrying out an antigen-antibody reaction using a specific antibody to that certain molecule. The detection methods are broadly classified into direct methods and indirect methods, and examples of indirect methods include enzyme antibody methods, fluorescent antibody methods, avidin-biotin peroxidase complex (ABC) methods, and gold colloid methods. Examples of samples to be stained include cells; cellulose acetate membranes, polyacrylamide gel, or agarose gel following electrophoresis; and nitrocellulose membranes, Nylon membranes, or polyvinylidene difluoride (PVDF) membranes transferred by blotting methods such as western blotting, southern blotting, or northern blotting.

The metal complex compound represented by general formula (I) or the zinc complex compound represented by general formula (II) can be used as a solution by dissolving in a solvent. There are no particular limitations on the solvent that can be used provided it does not impair detection of substances having a phosphate group, and examples include water (including buffers and other salt solutions), alcohols such as methanol or ethanol, and mixed solvents thereof, with aqueous mixed solvents such as water (including buffers and other salt solutions) and methanol or water (including buffers and other salt solutions) and ethanol being preferable.

The following describes examples of a method for staining a substance having a phosphate group as claimed in the present invention.

First, an example is indicated of a method for staining a phosphorylated protein. A sample is prepared from the target cell tissue that contains essentially all of the proteins that compose the cell. This preparation can be carried out by a method ordinarily used in the field of biochemistry. Next, the protein contained in the sample is separated. There are no particular limitations on the separation method, and although a typical method can be used, electrophoresis, for example, is used preferably. In the case of using electrophoresis, the gel following electrophoresis, or a membrane to which the gel has been transferred following electrophoresis by western blotting, is immersed in a solution of the metal complex compound represented by general formula (I) or the zinc complex compound represented by general formula (II) to label the phosphorylated protein followed by identification of the phosphorylated protein using a staining method corresponding to the type of labeling group.

Next, an example is indicated of a method for staining deoxyribonucleic acid (DNA) or ribonucleic acid (RNA). In a membrane and the like transferred by southern blotting or northern blotting, a terminal phosphate group (phosphate monoester group) of deoxyribonucleic acid (DNA) or ribonucleic acid (RNA) complementary to a specific base sequence on a deoxyribonucleic acid (DNA) or ribonucleic acid (RNA) fragment is labeled by binding the metal complex compound represented by general formula (I) or the zinc complex compound represented by general formula (II) thereto to obtain a deoxyribonucleic acid (DNA) probe or ribonucleic acid (RNA) probe. Next, southern hybridization or northern hybridization is carried out using the deoxyribonucleic acid (DNA) probe or ribonucleic acid (RNA) probe, and a staining method corresponding to the type of labeling group is used to identify the deoxyribonucleic acid (DNA) or ribonucleic acid (RNA).

The following provides an explanation of an identifying method for a phosphorylated amino acid residue.

An "identifying method" refers to a method for identifying a phosphorylated amino acid residue in a phosphorylated protein or phosphorylated peptide, and by combining a commonly and frequently used immunostaining assay using an anti-phosphorylated amino acid antibody with the staining method for a substance having a phosphate group as the present invention, it is not only possible to distinguish between a phosphorylated protein and a non-phosphorylated protein, but also identify even a phosphorylated amino acid residue of the phosphorylated protein.

Examples of anti-phosphorylated amino acid antibodies used in the identifying method of the present invention include anti-phosphorylated serine antibody, anti-phosphorylated threonine antibody, and anti-phosphorylated tyrosine antibody.

In this identifying method, for example, only a phosphorylated protein is first detected among all proteins in a sample by labeling according to the method for staining a substance having a phosphate group as the present invention. Next, by carrying out an immunostaining assay using an anti-phosphorylated amino acid antibody to a certain phosphorylated amino acid and then comparing with the detection results of the above staining method, the phosphorylated protein can be determined to have the certain phosphorylated amino acid residue or have a phosphorylated amino acid residue other than the certain phosphorylated amino acid residue.

The metal complex compound in the present invention can be used as a staining agent or staining assistant for a substance having a phosphate group in the form of a solution in which the metal complex compound is dissolved in a suitable solvent. There are no particular limitations on the solvent that can be used provided it does not impair detection of the substance having a phosphate group, and examples include water (including buffers and other salt solutions), alcohols such as methanol or ethanol, and mixed solvents thereof, with aqueous mixed solvents such as water (including buffers and other salt solutions) and methanol or water (including buffers and other salt solutions) and ethanol being preferable.

A metal complex compound of the present invention in which the labeling group is an enzyme can be used in the form of a staining kit for a substance having a phosphate group by combining with a coloring agent corresponding to the enzyme or combining with a coloring agent and substrate corresponding to the enzyme. Examples of such coloring agents or combinations of coloring agents and substrates for enzymes include ECL (trademark), Super Signal (registered trademark), or combination of diaminobenzidine (DAB) and peroxidase for horseradish peroxidase (HRP); CDP-Star (trademark), CSPD (registered trademark), combination of nitro blue tetrazolium (NBT) and 5-bromo-4-chloro-3-indolyl phosphate, or AttoPhos (trademark) for alkaline phosphatase; and luciferin for luciferase. In addition, a metal complex compound of the present invention in which the labeling group is biotin can be used in the form of a staining kit for a substance having a phosphate group by combining with an enzyme bound with avidin, streptoavidin, or anti-biotin antibody and a coloring agent corresponding to the enzyme; or combining with an enzyme bound with avidin, streptoavidin, or anti-biotin antibody and a coloring agent and substrate corresponding to the enzyme.

### Example

Although the following provides a more detailed explanation of the present invention through examples thereof, the scope of the present invention is not limited thereto.

### Example 1

### Production of Zinc Complex Compound-Horseradish Peroxidase (HRP)-Bound Streptoavidin Complex Solution

10 mM N,N,N'-tris(2-pyridylmethyl)-N'-(2-D-biotinamidoethyl)carbamoyl-2-pyridylmethyl]-1,3-diamino-2-propanol represented by formula (IV):

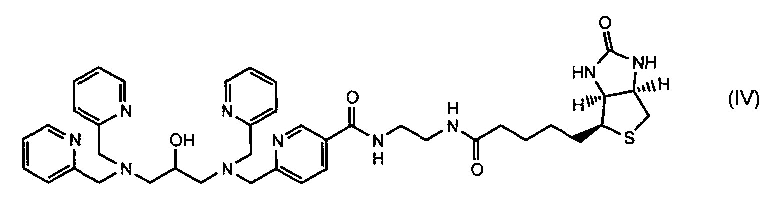

10 mM tris-hydrochloric acid buffer (pH 7.5), 100 mM sodium chloride, and 0.1% (w/v) Tween 20 were adjusted with a 10% (v/v) aqueous methanol solution to obtain a ligand solution. 100 mM tris-hydrochloric acid buffer (pH 7.5), 1 M sodium chloride, and 1% (w/v) Tween 20 were adjusted with water to obtain TBS-T (10X). 10 mM tris-hydrochloric acid buffer (pH 7.5), 100 mM sodium chloride, and 0.1% (w/v) Tween 20 were adjusted with water to obtain TBS-T (1X). 5 µL of the ligand solution, 50 µL of TBS-T (10X), 5 µL of 10 mM aqueous zinc nitrate solution, 1 µL of horseradish peroxidase (HRP)-bound streptoavidin, and 394 µL of water were mixed followed by ultrafiltration (30 kDa), and the residue was dissolved with 30 mL of TBS-T (1X) to obtain a solution of the zinc complex compound-horseradish peroxidase (HRP)-bound streptoavidin compound represented by formula (V).

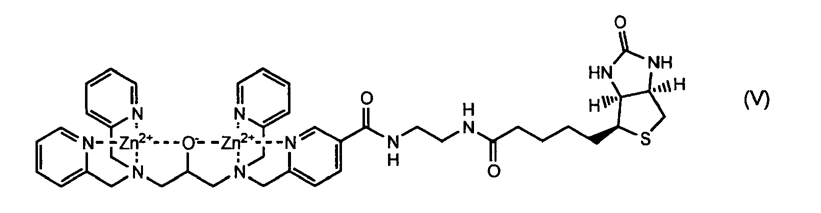

### Example 2

### Phosphorylated Protein Staining Method 1

Gels for SDS-polyacrylamide gel electrophoresis (SDS-PAGE), a phoresis tank buffer, and a sample preparation liquid were first prepared under the conditions indicated below.
Concentration Gel Solution:
   4.5% (w/v) polyacrylamide (acrylamide:bisacrylamide = 30:1)
   125 mM tris-hydrochloric acid buffer (pH 6.8)
   0.1% (w/v) sodium dodecyl sulfate (SDS)
Separation Gel Solution:
   12.5% (w/v) polyacrylamide (acrylamide:bisacrylamide = 30:1)
   375 mM tris-hydrochloric acid buffer (pH 8.8)
   0.1% (w/v) sodium dodecyl sulfate (SDS)
Phoresis Tank Buffer:
   25 mM Tris
   192 mM glycine
   0.1% (w/v) sodium dodecyl sulfate (SDS)
Sample Preparation Liquid:
   195 mM tris-hydrochloric acid buffer (pH 6.8)
   9% (w/v) sodium dodecyl sulfate (SDS)
   24% (w/v) glycerol
   15% (w/v) 2-mercaptoethanol
   0.1% (w/v) bromophenol blue (BPB)

Next, a molecular weight marker (trypsin inhibitor, carbonate dehydratase, ovalbumin, bovine serum albumin, and phosphorylase b) in lane 1, α-casein in lane 2, dephosphorylated α-casein in lane 3, β-casein in lane 4, and dephosphorylated β-casein in lane 5 were dissolved in sample preparation liquid for use as samples followed by plotting onto the prepared SDS-polyacrylamide gel electrophoresis (SDS-PAGE) gel and electrophoresing at a constant current of 40 mA until the bromophenol blue (BPB) reached the bottom of the gel.

The gel on which SDS-polyacrylamide gel electrophoresis (SDS-PAGE) was carried out was immersed in a fixing solution for about 15 minutes followed by immersing in SYPRO (registered trademark) Ruby protein gel stain for about 3 hours, taking out of the solution, washing with decoloring liquid for 2 hours, and visualizing with a UV transilluminator.

In addition, the protein on the gel on which SDS-polyacrylamide gel electrophoresis (SDS-PAGE) was carried out was transferred to a polyvinylidene difluoride (PVDF) membrane by semi-dry blotting. After immersing the transferred polyvinylidene difluoride (PVDF) membrane in the zinc complex compound-horseradish peroxidase (HRP)-bound streptoavidin complex solution prepared in Example 1 for about 30 minutes, the membrane was taken out of the solution and washed for about 10 minutes followed by visualizing with a chemiluminescent image analyzer using a coloring agent of ECL (trademark) Plus.

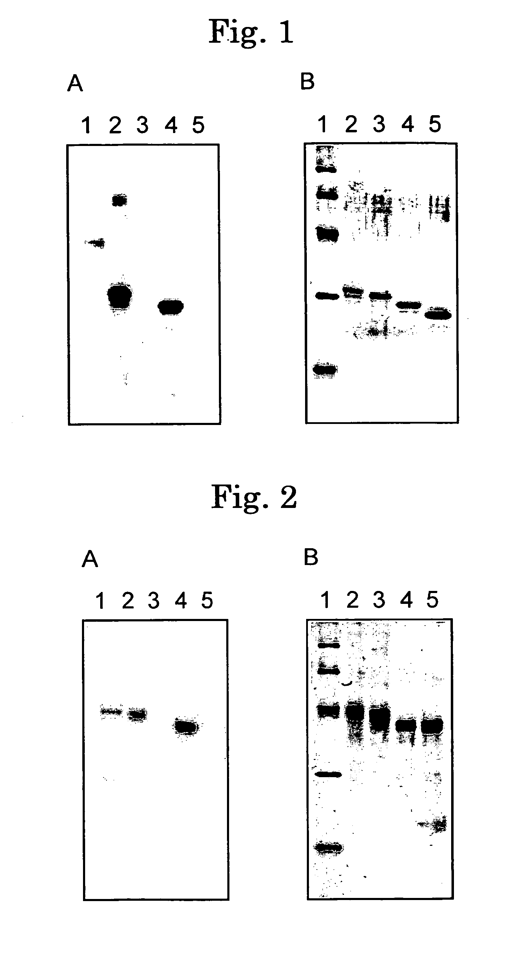

The polyvinylidene difluoride (PVDF) membrane following staining with the zinc complex compound-horseradish peroxidase (HRP)-bound streptoavidin complex solution as the present invention, and the gel following staining with SYPRO (registered trademark) Ruby protein gel stain, are shown in FIG. 1 as A and B, respectively.

As shown in FIG. 1, only α-casein and β-casein having phosphate groups can be identified according to the method of the present invention. Thus, according to the present invention, only phosphorylated protein was clearly demonstrated to be able to be identified from a biological sample.

### Example 3

### Phosphorylated Protein Staining Method 2

Gels for SDS-polyacrylamide electrophoresis (SDS-PAGE), a phoresis tank buffer and a sample preparation liquid were first prepared under the same conditions as the aforementioned Example 2.

Next, a molecular weight marker (trypsin inhibitor, carbonate dehydratase, ovalbumin, bovine serum albumin, and phosphorylase b) in lane 1, ovalbumin in lane 2, dephosphorylated ovalbumin in lane 3, pepsin in lane 4, and dephosphorylated pepsin in lane 5 were dissolved in sample preparation liquid for use as samples followed by plotting onto the prepared SDS-polyacrylamide gel electrophoresis (SDS-PAGE) gel and electrophoresing at a constant current of 40 mA until the bromophenol blue (BPB) reached the bottom of the gel.

The gel on which SDS-polyacrylamide gel electrophoresis (SDS-PAGE) was carried out was immersed in a fixing solution for about 15 minutes followed by immersing in SYPRO (registered trademark) Ruby protein gel stain for about 3 hours, taking out of the solution, washing with decoloring liquid for 2 hours, and visualizing with a UV transilluminator.

In addition, the protein on the gel on which SDS-polyacrylamide gel electrophoresis (SDS-PAGE) was carried out was transferred to a polyvinylidene difluoride (PVDF) membrane by semi-dry blotting. After immersing the transferred polyvinylidene difluoride (PVDF) membrane in the zinc complex compound-horseradish peroxidase (HRP)-bound streptoavidin complex solution prepared in Example 1 for about 30 minutes, the membrane was taken out of the solution and washed for about 10 minutes followed by visualizing with a chemiluminescent image analyzer using a coloring agent of ECL (trademark) Plus.

The polyvinylidene difluoride (PVDF) membrane following staining with the zinc complex compound-horseradish peroxidase (HRP)-bound streptoavidin complex solution as the present invention, and the gel following staining with SYPRO (registered trademark) Ruby protein gel stain, are shown in FIG. 2 as A and B, respectively.

As shown in FIG. 2, only ovalbumin and pepsin having phosphate groups can be identified according to the method of the present invention. Thus, according to the present invention, only phosphorylated protein was clearly demonstrated to be able to be identified from a biological sample.

### Example 4

### Phosphorylated Amino Acid Residue Identification Method

Gels for SDS-polyacrylamide electrophoresis (SDS-PAGE), a phoresis tank buffer and a sample preparation liquid were first prepared under the same conditions as the aforementioned Example 2.

Next, an A-431 whole cell lysate in lane 1, an epidermal growth factor (EGF)-stimulated A-431 whole cell lysate in lane 2, and an alkaline phosphatase-treated epidermal growth factor (EGF)-stimulated A-431 whole cell lysate in lane 3 were dissolved in sample preparation liquid for use as samples followed by plotting onto the prepared SDS-polyacrylamide gel electrophoresis (SDS-PAGE) gel and electrophoresing at a constant current of 40 mA until the bromophenol blue (BPB) reached the bottom of the gel.

The gel on which SDS-polyacrylamide gel electrophoresis (SDS-PAGE) was carried out was immersed in a fixing solution for about 15 minutes followed by immersing in SYPRO (registered trademark) Ruby protein gel stain for about 3 hours, taking out of the solution, washing with decoloring liquid for 2 hours, and visualizing with a UV transilluminator.

In addition, the protein on the gel on which SDS-polyacrylamide gel electrophoresis (SDS-PAGE) was carried out was transferred to a polyvinylidene difluoride (PVDF) membrane by semi-dry blotting. After immersing the transferred polyvinylidene difluoride (PVDF) membrane in the zinc complex compound-horseradish peroxidase (HRP)-bound streptoavidin complex solution prepared in Example 1 for about 30 minutes, the membrane was taken out of the solution and washed for 10 minutes followed by visualizing with a chemiluminescent image analyzer using a coloring agent of ECL (trademark) Plus.

In addition, after immersing the transferred polyvinylidene difluoride (PVDF) membrane in 1% bovine serum albumin TBS-T (1X) solution for about 1 hour, the membrane was taken out of the solution and washed for 20 minutes, and then immersed in 0.1% horseradish peroxidase (HRP)-bound anti-phosphorylated tyrosine antibody TBS-T (1X) solution for about 1 hour, taken out of the solution and washed for 20 minutes, followed by visualizing with a chemiluminescent image analyzer using a coloring agent of ECL (trademark) Plus.

In addition, after immersing the transferred polyvinylidene difluoride (PVDF) membrane in 1% bovine serum albumin TBS-T (1X) solution for about 1 hour, the membrane was taken out of the solution and washed for 20 minutes, immersed in 0.1% rabbit anti-phosphorylated serine antibody TBS-T (1X) solution for about 1 hour, taken out of the solution and washed for 20 minutes, and then immersed in 0.008% horseradish peroxidase (HRP)-bound anti-rabbit immunoglobulin G (IgG) antibody TBS-T (1X) solution for about 1 hour, taken out of the solution and washed for 20 minutes, followed by visualizing with a chemiluminescent image analyzer using a coloring agent of ECL (trademark) Plus.

The polyvinylidene difluoride (PVDF) membrane following staining with the zinc complex compound-horseradish peroxidase (HRP)-bound streptoavidin complex solution as the present invention, the polyvinylidene difluoride (PVDF) membrane following staining with the horseradish peroxidase (HRP)-bound anti-phosphorylated tyrosine antibody solution, the polyvinylidene difluoride (PVDF) membrane following staining with the rabbit anti-phosphorylated serine antibody solution and horseradish peroxidase (HRP)-bound anti-rabbit immunoglobulin G (IgG) antibody solution, and the gel following staining with SYPRO (registered trademark) Ruby protein gel stain, are shown in FIG. 3 as A, B, C, and D, respectively.

As shown in FIG. 3, phosphorylated protein and phosphorylated protein having a phosphorylated tyrosine residue, phosphorylated protein having a phosphorylated serine residue and phosphorylated proteins having other phosphorylated amino acid residues were able to be identified in A-431 whole cell lysate, epidermal growth factor (EGF)-stimulated A-431 whole cell lysate, and alkaline phosphatase-treated epidermal growth factor (EGF)-stimulated A-431 whole cell lysate by the method of the present invention. Thus, according to the present invention, phosphorylated protein and phosphorylated amino acid residues were clearly demonstrated to be able to be identified in biological samples.

### INDUSTRIAL APPLICABILITY

The staining agent and staining method for substances having a phosphate group as the present invention enable substances having a phosphate group to be easily detected. Thus, application of the present invention to a biological sample and the like is extremely useful in terms of being able to be applied to disease diagnosis and the like. In addition, combining the staining method for substances having a phosphate group as the present invention with anti-phosphorylated amino acid antibody enables phosphorylated amino acid residues to be easily identified. In addition, since a compound as the present invention demonstrates coordinate bonding unlike that found in the prior art with substances having a phosphate group, it is useful as a compound able to be used in the aforementioned method.

## Claims

1. A method for staining a substance having a phosphate group, **characterized by** using a metal complex compound represented by general formula (I): 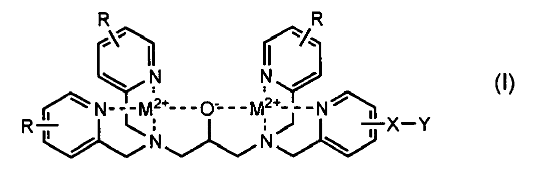 (wherein, M represents a metal atom capable of becoming a dication, each R may be identical or different from each other and represents either a hydrogen atom; an alkyl group of 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group (wherein, the alkyl part thereof is of 1 to 16 carbon atoms); a carboxyl group; a carbamoyl group; a cyano group; a hydroxyl group; an amino group; or a halogeno group, X represents a binding group, and Y represents a labeling group).

2. A method for staining a substance having a phosphate group, **characterized by** using a zinc complex compound represented by general formula (II): 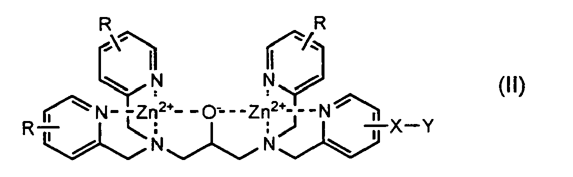 (wherein, each R may be identical or different from each other and represents either a hydrogen atom; an alkyl group of 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group (wherein, the alkyl part thereof is of 1 to 16 carbon atoms); a carboxyl group; a carbamoyl group; a cyano group; a hydroxyl group; an amino group; or a halogeno group, X represents a binding group, and Y represents a labeling group).

3. The method for staining a substance having a phosphate group according to claim 1 or 2, wherein the labeling group is biotin.

4. A method for identifying a phosphorylated amino acid residue, **characterized by** combining the method for staining a substance having a phosphate group according to claim 1 with an immunostaining assay using an anti-phosphorylated amino acid antibody.

5. A method for identifying a phosphorylated amino acid residue, **characterized by** combining the method for staining a substance having a phosphate group according to claim 2 with an immunostaining assay using an anti-phosphorylated amino acid antibody.

6. A method for identifying a phosphorylated amino acid residue, **characterized by** combining the method for staining a substance having a phosphate group according to claim 3 with an immunostaining assay using an anti-phosphorylated amino acid antibody.

7. A metal complex compound represented by general formula (III): 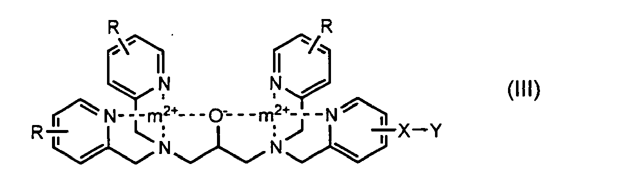 (wherein, m represents a metal atom capable of becoming a dication (excluding the case in which m is a zinc atom), each R may be identical or different from each other and represents either a hydrogen atom; an alkyl group of 1 to 16 carbon atoms; an acyl group, an alkoxycarbonyl group, an acylalkyl group, an alkoxycarbonylalkyl group, a carboxyalkyl group, a carbamoylalkyl group, a cyanoalkyl group, a hydroxyalkyl group, an aminoalkyl group, or a haloalkyl group (wherein, the alkyl part thereof is of 1 to 16 carbon atoms); a carboxyl group; a carbamoyl group; a cyano group; a hydroxyl group; an amino group; or a halogeno group, X represents a binding group, and Y represents a labeling group).

8. The metal complex compound according to claim 7, wherein the labeling group is biotin.

9. A staining agent or a staining assistant for a substance having a phosphate group, **characterized by** comprising the metal complex compound represented by general formula (I) according to claim 1.

10. A staining kit for a substance having a phosphate group, **characterized by** comprising the metal complex compound represented by general formula (I) according to claim 1 (wherein, Y is an enzyme), a coloring agent for the enzyme, and a substrate as necessary.

11. A staining kit for a substance having a phosphate group, **characterized by** comprising the metal complex compound represented by general formula (I) according to claim 1 (wherein, Y is biotin); an enzyme bound by avidin, streptoavidin, or anti-biotin antibody; a coloring agent for the enzyme; and a substrate as necessary.
